# EUROPEAN PATENT SPECIFICATION

(11) **EP 4 422 508 B1**
(45) Date of publication and mention of the grant of the patent: **16.09.2026**
(21) Application number: 22835158.1
(22) Date of filing: 02.11.2022
(51) Int. Cl.: A61B 8/08, A61B 8/00, A61B 8/06

(54) **DOPPLER-BASED VEIN-ARTERY DETECTION FOR VASCULAR ASSESSMENT**
DOPPLER-BASIERTE VENENARTERIENDETEKTION ZUR GEFÄSSBEURTEILUNG
DÉTECTION D'ARTERE VEINEUX À BASE DE DOPPLER POUR ÉVALUATION VASCULAIRE

(30) Priority: 03.11.2021 US 202163275223 P
(43) Date of publication of application: 04.09.2024
(73) Proprietor: Bard Access Systems, Inc., Salt Lake City, UT 84116 (US)
(72) Inventor: MISENER, Anthony K., Bountiful, UT 84010 (US); SOWARDS, Steffan, Salt Lake City, UT 84124 (US); MCLAUGHLIN, William Robert, Bountiful, UT 84010 (US)
(74) Representative: Hoffmann Eitle
(86) International application number: PCT/US2022/048716
(87) International publication number: WO 2023/081220

(56) References cited:
- WO-A1-2010/076808
- WO-A1-2021/055289
- US-A1- 2021 137 492
- US-A1- 2021 315 538

## Description

### BACKGROUND

Ultrasound imaging is a widely accepted tool for guiding interventional instruments such as needles to targets such as blood vessels or organs in the human body. In order to successfully guide, for example, a needle to a blood vessel using ultrasound imaging, the needle is monitored in real-time both immediately before and after a percutaneous puncture in order to enable a clinician to determine the distance and the orientation of the needle to the blood vessel and ensure successful access thereto. However, through inadvertent movement of an ultrasound probe during the ultrasound imaging, the clinician can lose both the blood vessel and the needle, which can be difficult and time consuming to find again. In addition, it is often easier to monitor the distance and orientation of the needle immediately before the percutaneous puncture with a needle plane including the needle perpendicular to an image plane of the ultrasound probe. And it is often easier to monitor the distance and orientation of the needle immediately after the percutaneous puncture with the needle plane parallel to the image plane. As with inadvertently moving the ultrasound probe, the clinician can lose both the blood vessel and the needle when adjusting the image plane before and after the percutaneous puncture, which can be difficult and time consuming to find again. What is needed are ultrasound-imaging systems and methods thereof that can dynamically adjust the image plane to facilitate guiding interventional instruments to targets in at least the human body.

Doppler ultrasound is a noninvasive approach to estimating the blood flow through your blood vessels by bouncing high-frequency sound waves (ultrasound) off circulating red blood cells. A doppler ultrasound can estimate how fast blood flows by measuring the rate of change in its pitch (frequency). During a doppler ultrasound, a technician trained in ultrasound imaging (sonographer) positions an ultrasound probe against the skin over a predefined area. Doppler ultrasound may be performed as an alternative to more-invasive procedures, such as angiography, which involves injecting dye into the blood vessels so that they show up clearly on X-ray images.

A Doppler ultrasound may help diagnose many conditions, including blood clots, poorly functioning valves in your leg veins, which can cause blood or other fluids to pool in your legs (venous insufficiency), heart valve defects and congenital heart disease, a blocked artery (arterial occlusion), decreased blood circulation into your legs (peripheral artery disease), bulging arteries (aneurysms), and narrowing of an artery, such as in your neck (carotid artery stenosis)

Doppler ultrasound may also detect a direction of blood flow. For example, doppler ultrasound can differentiate an artery from a vein since the direction of blood flow within an artery is generally in the opposite direction from a blood flow within an adjacent vein. As doppler ultrasound applies to blood flow, ultrasound images that include doppler ultrasound commonly portray results in a manner that obstructs the visibility of the blood vessel thereby causing difficulty in visualizing an image of a medical device in relation to the blood vessel.

Disclosed herein are systems and methods providing visual notifications pertaining to doppler ultrasound that for identifying a blood vessel within an ultrasound image based on doppler ultrasound and providing visual notification pertaining to doppler ultrasound results that maintain visibility of the cross-sectional area of the blood vessel.

US 2021/137492 A1 discloses an ultrasound diagnostic apparatus including an ultrasound probe, an image acquisition unit that transmits an ultrasound beam toward a subject from the ultrasound probe to sequentially acquire ultrasound images, a blood vessel detection unit that detects a blood vessel included in the ultrasound image acquired by the image acquisition unit, a blood vessel discrimination unit that discriminates whether the blood vessel detected by the blood vessel detection unit is a vein or an artery, and a discrimination execution deciding unit that decides whether the blood vessel discrimination unit newly executes discrimination with respect to the ultrasound image of a current frame based on a movement amount of the ultrasound probe or a change amount of the ultrasound image between frames.

US 2021/315538 A1 discloses methods and systems for ultrasound imaging. A method comprises acquiring, via an ultrasound probe, Doppler measurements over a plurality of cardiac cycles, evaluating a flow profile comprising the Doppler measurements to detect an abnormality in the flow profile, and displaying, via a display device, the flow profile with a reference flow profile overlaid thereon.

WO 2010/076808 A1 discloses an integrated ultrasound imaging device with pulse oximeter waveform display for application of regional anesthesia. The device has an ultrasound probe and a Saturation of Hemoglobin with Oxygen as measured by Pulse Oximetry (SpO2) prob connected to a display unit through a beam former and a pulse oximeter respectively. A processor is connected to the ultrasound probe, the SpO2 probe and the display unit to receive the output signals from the ultrasound probe and the SpO2 probe to display a real time ultrasound image of the scanned area and the measured SpO2 values on the same display unit. It provides a color doppler imaging mode to differentiate between the nerves and blood vessels to avoid puncture the blood vessel.

WO 2021/055289 A1 discloses an ultrasound medical imaging system that detects blood flow within a blood vessel and classifies the flow using a color, a pattern or a label.

### SUMMARY

Disclosed herein is an ultrasound-imaging system including, in some embodiments, an ultrasound probe, a console, and a display screen. The ultrasound probe includes an array of ultrasonic transducers, where activated ultrasonic transducers of the array of ultrasonic transducers are configured to emit generated ultrasound signals into a patient, receive reflected ultrasound signals from the patient, and convert the reflected ultrasound signals into corresponding electrical signals of the ultrasound signals for processing into ultrasound image data and doppler ultrasound data. The console is configured to communicate with the ultrasound probe, and the console includes one or more processors and a non-transitory computer-readable medium having stored thereon logic that, when executed by the one or more processors, causes system operations.

The operations include: (i) detecting a black hole within a predefined target area of the patient based on ultrasound image data, (ii) determining a blood flow condition within the black hole based at least partially on doppler ultrasound data, (iii) defining an ultrasound image of the predefined target area including an image of the black hole, and (iv) superimposing a notification atop the ultrasound image, where the notification indicates the direction of blood flow with respect to the image of the black hole,

In some embodiments, the operations further include determining a direction of blood flow within the black hole with respect to an image plane of the ultrasound image, and the notification indicates the direction of blood flow with respect to the image of the black hole

In some embodiments, the operations further include rendering the ultrasound image on a display coupled with the system.

In some embodiments, the notification does not obstruct the image of the black hole. In further embodiments, the operations further include defining a boundary surrounding the image of the black hole and superimposing the notification outside of the boundary.

The operations my further include identifying the blood vessel as a vein or alternatively as an artery, where identifying the blood vessel as a vein or an artery is based at least partially on the condition of the blood flow within the blood vessel.

The operations my further include identifying the blood vessel as a vein or alternatively as an artery, based at least partially on the direction of the blood flow with respect to the image plane.

In some embodiments, the doppler ultrasound data includes a measurement of a pulsatility of the blood flow, and identifying the blood vessel as a vein or an artery is based at least partially on the pulsatility measurement.

In some embodiments, the operations further include: (i) comparing the ultrasound image with one or more corresponding ultrasound images stored in memory, where the comparison including a comparison of spatial positioning of the black hole within the ultrasound image, and (ii) at least partially as a result of the comparison, identifying the blood vessel as a vein or an artery. The notification may indicate the identity of the blood vessel as a vein or an artery and the notification may further indicate a confidence for the identification of the blood vessel.

In some embodiments, the operations further include measuring a blood flow rate within the black hole, comparing the blood flow rate with a range of blood flow rates stored in memory and as a result of the comparison, determining that the blood flow rate within the blood vessel is compromised. The operations may further include, as a result of the comparison, determining that the blood vessel is partially and/or totally occluded.

In some embodiments, the operations further include: (i) obtaining a first blood flow rate measurement for the blood vessel, (ii) obtaining a second blood flow rate measurement for the blood vessel, the second measurement subsequent to the first measurement, (iii) comparing the second measurement with the first measurement, and (iv) as a result of the flow rate measurement comparison, determining that the blood flow rate within the blood vessel is compromised.

In some embodiments, the operations further include detecting a plurality of black holes within the predefined target area of the patient based on ultrasound image data, determining a blood flow condition within each of the black holes based at least partially on doppler ultrasound data, and identifying each of the black holes as a blood vessel or alternatively as one or more nerves, where identifying the black hole as one or more nerves includes determining a non-flow condition of blood within the respective black hole.

The operations may further include defining a doppler ultrasound window extending at least partially across the ultrasound image and determining the blood flow condition within each of the one or more black holes encompassed by the doppler ultrasound window. In some embodiments, defining the doppler ultrasound window includes detecting the one or more black holes based on the ultrasound image data, and automatically defining the doppler ultrasound window to encompass the one or more black holes.

In some embodiments, defining the doppler ultrasound window includes receiving an input via an input device of the system, and defining the doppler ultrasound window based on the input, where the input includes a selected portion of the ultrasound image.

In some embodiments, the ultrasound probe further includes an array of magnetic sensors configured to convert magnetic signals from a magnetized medical device into corresponding electrical signals of the magnetic signals for processing by the processor into distance and orientation information with respect to the predefined target area for rendering of an iconographic representation of the medical device atop the ultrasound image. I n further embodiments, defining the doppler ultrasound window includes defining the doppler ultrasound window based on a position of the iconographic representation of the medical device atop the ultrasound image.

Disclosed herein also is method of an ultrasound-imaging system including a non-transitory computer-readable medium ("CRM") having executable logic that cause the ultrasound-imaging system to perform a set of operations for ultrasound imaging when the logic is executed by a processor of a console of the ultrasound-imaging system, where the method includes performing the operations described above.

These and other features of the concepts provided herein will become more apparent to those of skill in the art in view of the accompanying drawings and following description, which describe particular embodiments of such concepts in greater detail.

### DRAWINGS

FIG. 1 illustrates an ultrasound-imaging system and a patient in accordance with some embodiments.
FIG. 2 illustrates a block diagram of a console of the ultrasound-imaging system of FIG. 1 in accordance with some embodiments.
FIG. 3A illustrates an ultrasound probe of the ultrasound-imaging system imaging a blood vessel in accordance with some embodiments.
FIG. 3B illustrates an ultrasound image of the blood vessel of FIG. 3A on a display screen of the ultrasound-imaging system in accordance with some embodiments.
FIG. 4 illustrates the ultrasound probe of the ultrasound-imaging system configured as a 2-D ultrasound probe in accordance with some embodiments.
FIG. 5A illustrates an ultrasound image including black holes in accordance with some embodiments.
FIG. 5B illustrates an ultrasound image of FIG. 5A further including a doppler ultrasound window in accordance with some embodiments.
FIG. 5C illustrates an ultrasound image of FIG. 5A indicating blood flow through the black holes in accordance with some embodiments.
FIG. 5D illustrates an ultrasound image similar to the ultrasound image of FIG. 5C where blood flow through the black holes is compromised in accordance with some embodiments.
FIG. 6 illustrates an exemplary screenshot of the ultrasound image of the FIG. 5A including notifications superimposed atop the ultrasound in accordance with some embodiments.
FIG. 7 is a flow chart illustrating operations of a method performed by the system of FIG. 1 in accordance with some embodiments.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal-end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal-end portion, or the proximal length of the catheter can include the proximal end of the catheter; however, the proximal portion, the proximal-end portion, or the proximal length of the catheter need not include the proximal end of the catheter. That is, unless context suggests otherwise, the proximal portion, the proximal-end portion, or the proximal length of the catheter is not a terminal portion or terminal length of the catheter.

With respect to "distal," a "distal portion" or a "distal-end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal-end portion, or the distal length of the catheter can include the distal end of the catheter; however, the distal portion, the distal-end portion, or the distal length of the catheter need not include the distal end of the catheter. That is, unless context suggests otherwise, the distal portion, the distal-end portion, or the distal length of the catheter is not a terminal portion or terminal length of the catheter.

Unless defined otherwise, all technical and scientific terms used herein have the same meaning as commonly understood by those of ordinary skill in the art.

### Ultrasound-imaging systems

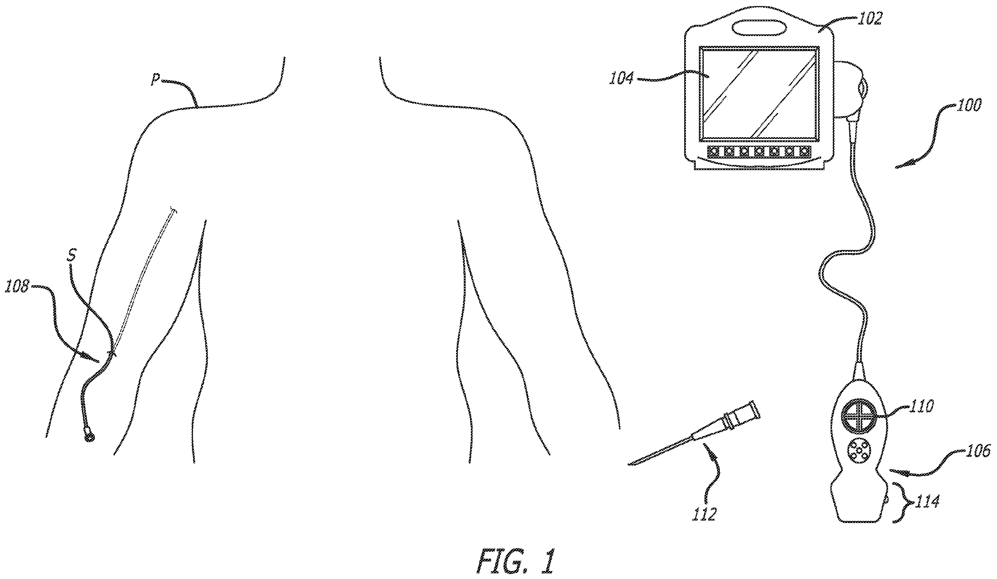

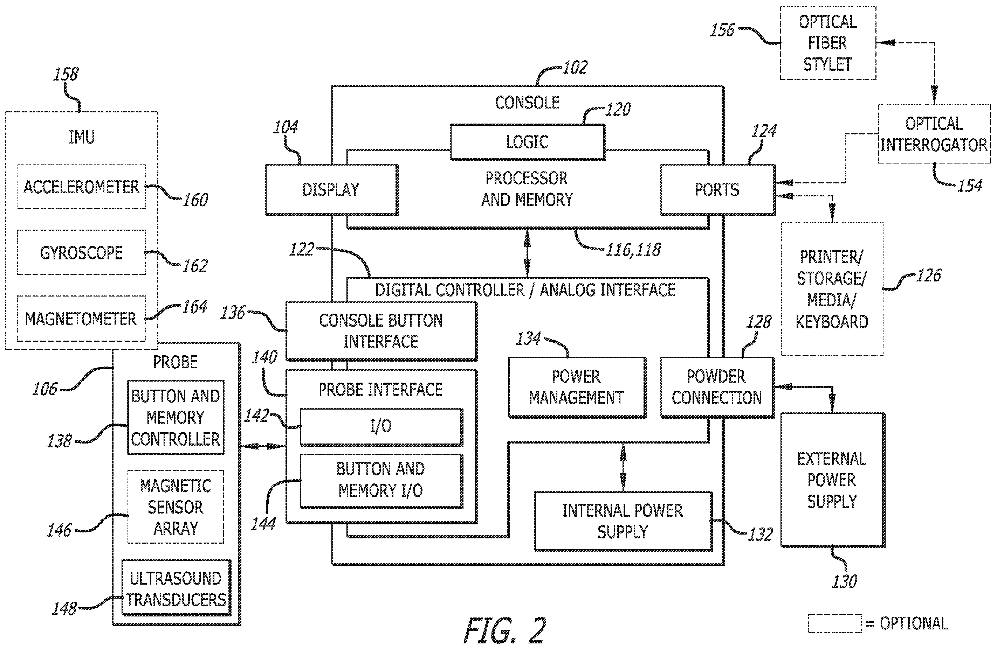

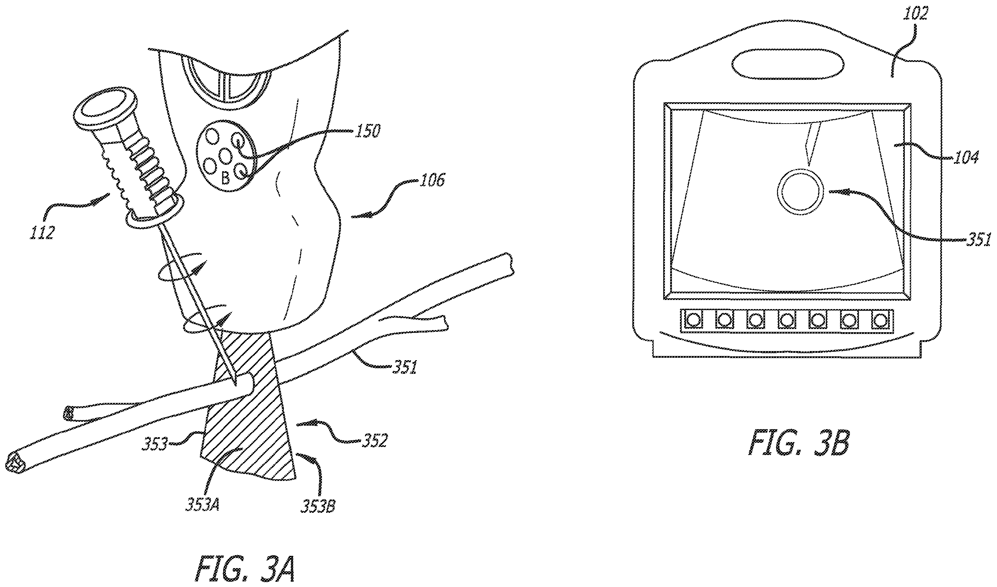

FIG. 1 illustrates an ultrasound-imaging system 100, a needle 112, and a patient *P* in accordance with some embodiments. FIG. 2 illustrates a block diagram of the ultrasound-imaging system 100 in accordance with some embodiments. FIG. 3A illustrates an ultrasound probe 106 of the ultrasound-imaging system 100 imaging a blood vessel of the patient *P* prior to accessing the blood vessel in accordance with some embodiments. FIG. 3B illustrates an ultrasound image of the blood vessel of FIG. 3A on a display screen 104 of the ultrasound-imaging system 100 with an iconographic representation of the needle 112 in accordance with some embodiments.

As shown, the ultrasound-imaging system 100 includes a console 102, the display screen 104, and the ultrasound probe 106. The ultrasound-imaging system 100 is useful for imaging a target such as a blood vessel or an organ within a body of the patient *P* prior to a percutaneous puncture with the needle 112 for inserting the needle 112 or another medical device into the target and accessing the target. Indeed, the ultrasound-imaging system 100 is shown in FIG. 1 in a general relationship to the patient *P* during an ultrasound-based medical procedure to place a catheter 108 into the vasculature of the patient *P* through a skin insertion site *S* created by a percutaneous puncture with the needle 112. It should be appreciated that the ultrasound-imaging system 100 can be useful in a variety of ultrasound-based medical procedures other than catheterization. For example, the percutaneous puncture with the needle 112 can be performed to biopsy tissue of an organ of the patient *P.*

The console 102 houses a variety of components of the ultrasound-imaging system 100, and it is appreciated the console 102 can take any of a variety of forms. A processor 116 and memory 118 such as random-access memory ("RAM") or non-volatile memory (e.g., electrically erasable programmable read-only memory ["EEPROM"]) is included in the console 102 for controlling functions of the ultrasound-imaging system 100, as well as executing various logic operations or algorithms during operation of the ultrasound-imaging system 100 in accordance with executable logic 120 therefor stored in the memory 118 for execution by the processor 116. For example, the console 102 is configured to instantiate by way of the logic 120 one or more processes for dynamically adjusting a distance of activated ultrasonic transducers 149 from a predefined target area (e.g., an area including a blood vessel), an orientation of the activated ultrasonic transducers 149 to the predefined target area, or both the distance and the orientation of the activated ultrasonic transducers 149 with respect to the predefined target area, as well as process electrical signals from the ultrasound probe 106 into ultrasound images. Dynamically adjusting the activated ultrasonic transducers 149 uses ultrasound-imaging data, magnetic-field data, shape-sensing data, or a combination thereof received by the console 102 for activating certain ultrasonic transducers of a 2-D array of the ultrasonic transducers 148 or moving those already activated in a linear array of the ultrasonic transducers 148. A digital controller/analog interface 122 is also included with the console 102 and is in communication with both the processor 116 and other system components to govern interfacing between the ultrasound probe 106 and other system components set forth herein.

The ultrasound-imaging system 100 further includes ports 124 for connection with additional components such as optional components 126 including a printer, storage media, keyboard, etc. The ports 124 can be universal serial bus ("USB") ports, though other types of ports can be used for this connection or any other connections shown or described herein. A power connection 128 is included with the console 102 to enable operable connection to an external power supply 130. An internal power supply 132 (e.g., a battery) can also be employed either with or exclusive of the external power supply 130. Power management circuitry 134 is included with the digital controller/analog interface 122 of the console 102 to regulate power use and distribution.

Optionally, a stand-alone optical interrogator 154 can be communicatively coupled to the console 102 by way of one of the ports 124. Alternatively, the console 102 can include an integrated optical interrogator integrated into the console 102. Such an optical interrogator is configured to emit input optical signals into a companion optical-fiber stylet 156 for shape sensing with the ultrasound-imaging system 100, which optical-fiber stylet 156, in turn, is configured to be inserted into a lumen of a medical device such as the needle 112, and convey the input optical signals from the optical interrogator 154 to a number of FBG sensors along a length of the optical-fiber stylet 156. The optical interrogator 154 is also configured to receive reflected optical signals conveyed by the optical-fiber stylet 156 reflected from the number of FBG sensors, the reflected optical signals indicative of a shape of the optical-fiber stylet 156. The optical interrogator 154 is also configured to convert the reflected optical signals into corresponding electrical signals for processing by the console 102 into distance and orientation information with respect to the target for dynamically adjusting a distance of the activated ultrasonic transducers 149, an orientation of the activated ultrasonic transducers 149, or both the distance and the orientation of the activated ultrasonic transducers 149 with respect to the target or the medical device when it is brought into proximity of the target. For example, the distance and orientation of the activated ultrasonic transducers 149 can be adjusted with respect to a blood vessel as the target. Indeed, an image plane can be established by the activated ultrasonic transducers 149 being perpendicular or parallel to the blood vessel in accordance with an orientation of the blood vessel. The distance and orientation information can also be used for displaying an iconographic representation of the medical device on the display.

The display screen 104 is integrated into the console 102 to provide a GUI and display information for a clinician during such as one-or-more ultrasound images of the target area of the patient *P* attained by the ultrasound probe 106. In addition, the ultrasound-imaging system 100 enables the distance and orientation of a magnetized medical device such as the needle 112 to be superimposed in real-time atop an ultrasound image of the target, thus enabling a clinician to accurately guide the magnetized medical device to an intended target. Notwithstanding the foregoing, the display screen 104 can alternatively be separate from the console 102 and communicatively coupled thereto. A console button interface 136 and control buttons 110 (*see* FIG. 1) included on the ultrasound probe 106 can be used to immediately call up a desired mode to the display screen 104 by the clinician for assistance in an ultrasound-based medical procedure. In some embodiments, the display screen 104 is an LCD device.

The ultrasound probe 106 is employed in connection with ultrasound-based visualization of a target such as a blood vessel (*see* FIG. 3A) in preparation for inserting the needle 112 or another medical device into the target. Such visualization gives real-time ultrasound guidance and assists in reducing complications typically associated with such insertion, including inadvertent arterial puncture, hematoma, pneumothorax, etc. As described in more detail below, the ultrasound probe 106 is configured to provide to the console 102 electrical signals corresponding to both the ultrasound-imaging data, the magnetic-field data, the shape-sensing data, or a combination thereof for the real-time ultrasound guidance.

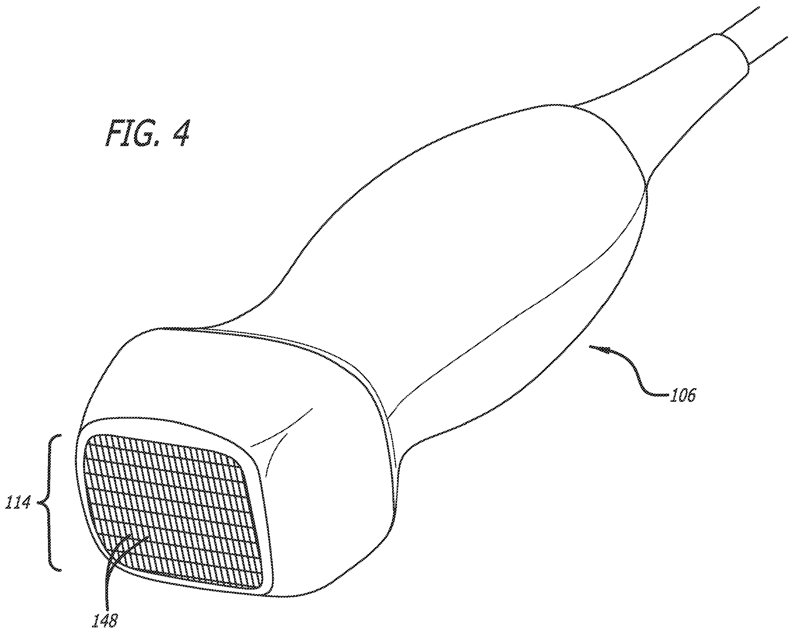

FIG. 4 illustrates the ultrasound probe 106 of the ultrasound-imaging system 100 configured as a 2-D ultrasound probe 106 in accordance with some embodiments. The ultrasound probe 106 includes a probe head 114 that houses a mounted and moveable (e.g., translatable or rotatable along a central axis) linear array of the ultrasonic transducers 148 or a 2-D array of the ultrasonic transducers 148, wherein the ultrasonic transducers 148 are piezoelectric transducers or capacitive micromachined ultrasonic transducers ("CMUTs"). When the ultrasound probe 106 is configured with the 2-D array of the ultrasonic transducers 148, a subset of the ultrasonic transducers 148 is linearly activated as needed for ultrasound imaging in accordance with ultrasound-imaging data, magnetic-field data, shape-sensing data, or a combination thereof to maintain the target in an image plane or switch to a different image plane (e.g., from perpendicular to a medical-device plane to parallel to the medical-device plane) including the target.

The probe head 114 is configured for placement against skin of the patient *P* proximate a prospective needle-insertion site where the activated ultrasonic transducers 149 in the probe head 114 can generate and emit the generated ultrasound signals into the patient *P* in a number of pulses, receive reflected ultrasound signals or ultrasound echoes from the patient *P* by way of reflection of the generated ultrasonic pulses by the body of the patient *P,* and convert the reflected ultrasound signals into corresponding electrical signals for processing into ultrasound images by the console 102 to which the ultrasound probe 106 is communicatively coupled. In this way, a clinician can employ the ultrasound-imaging system 100 to determine a suitable insertion site and establish vascular access with the needle 112 or another medical device.

The ultrasound probe 106 further includes the control buttons 110 for controlling certain aspects of the ultrasound-imaging system 100 during an ultrasound-based medical procedure, thus eliminating the need for the clinician to reach out of a sterile field around the patient *P* to control the ultrasound-imaging system 100. For example, a control button of the control buttons 110 can be configured to select or lock onto the target (e.g., a blood vessel, an organ, etc.) when pressed for visualization of the target in preparation for inserting the needle 112 or another medical device into the target. Such a control button can also be configured to deselect the target, which is useful whether the target was selected by the control button or another means such as by holding the ultrasound probe 106 stationary over the target to select the target, issuing a voice command to select the target, or the like.

FIG. 2 shows that the ultrasound probe 106 further includes a button and memory controller 138 for governing button and ultrasound probe 106 operation. The button and memory controller 138 can include non-volatile memory (e.g., EEPROM). The button and memory controller 138 is in operable communication with a probe interface 140 of the console 102, which includes an input/output ("I/O") component 142 for interfacing with the ultrasonic transducers 148 and a button and memory I/O component 144 for interfacing with the button and memory controller 138.

Also as seen in FIGS. 2 and 3A, the ultrasound probe 106 can include a magnetic-sensor array 146 for detecting a magnetized medical device such as the needle 112 during ultrasound-based medical procedures. The magnetic-sensor array 146 includes a number of magnetic sensors 150 embedded within or included on a housing of the ultrasound probe 106. The magnetic sensors 150 are configured to detect a magnetic field or a disturbance in a magnetic field as magnetic signals associated with the magnetized medical device when it is in proximity to the magnetic-sensor array 146. The magnetic sensors 150 are also configured to convert the magnetic signals from the magnetized medical device (e.g., the needle 112) into electrical signals for the console 102 to process into distance and orientation information for the magnetized medical device with respect to the predefined target, as well as for display of an iconographic representation of the magnetized medical device on the display screen 104. (*See* the magnetic field **B** of the needle 112 in FIG. 3A.) Thus, the magnetic-sensor array 146 enables the ultrasound-imaging system 100 to track the needle 112 or the like.

Though configured here as magnetic sensors, it is appreciated that the magnetic sensors 150 can be sensors of other types and configurations. Also, though they are described herein as included with the ultrasound probe 106, the magnetic sensors 150 of the magnetic-sensor array 146 can be included in a component separate from the ultrasound probe 106 such as a sleeve into which the ultrasound probe 106 is inserted or even a separate handheld device. The magnetic sensors 150 can be disposed in an annular configuration about the probe head 114 of the ultrasound probe 106, though it is appreciated that the magnetic sensors 150 can be arranged in other configurations, such as in an arched, planar, or semi-circular arrangement.

Each magnetic sensor of the magnetic sensors 150 includes three orthogonal sensor coils for enabling detection of a magnetic field in three spatial dimensions. Such 3-dimensional ("3-D") magnetic sensors can be purchased, for example, from Honeywell Sensing and Control of Morristown, NJ. Further, the magnetic sensors 150 are configured as Hall-effect sensors, though other types of magnetic sensors could be employed. Further, instead of 3-D sensors, a plurality of 1-dimensional ("1-D") magnetic sensors can be included and arranged as desired to achieve 1-, 2-, or 3-D detection capability.

Five magnetic sensors 150 are included in the magnetic-sensor array 146 so as to enable detection of a magnetized medical device such as the needle 112 in three spatial dimensions (e.g., X, Y, Z coordinate space), as well as the pitch and yaw orientation of the magnetized medical device itself. Detection of the magnetized medical device in accordance with the foregoing when the magnetized medical device is brought into proximity of the ultrasound probe 106 allows for dynamically adjusting a distance of the activated ultrasonic transducers 149, an orientation of the activated ultrasonic transducers 149, or both the distance and the orientation of the activated ultrasonic transducers 149 with respect to the target or the magnetized medical device. For example, the distance and orientation of the activated ultrasonic transducers 149 can be adjusted with respect to a blood vessel as the target. Indeed, an image plane can be established by the activated ultrasonic transducers 149 being perpendicular or parallel to the blood vessel in accordance with an orientation of the blood vessel. Note that in some embodiments, orthogonal sensing components of two or more of the magnetic sensors 150 enable the pitch and yaw attitude of the magnetized medical device to be determined, which enables tracking with relatively high accuracy. In other embodiments, fewer than five or more than five magnetic sensors of the magnetic sensors 150 can be employed in the magnetic-sensor array 146. More generally, it is appreciated that the number, size, type, and placement of the magnetic sensors 150 of the magnetic-sensor array 146 can vary from what is explicitly shown here.

As shown in FIG. 2, the ultrasound probe 106 can further include an inertial measurement unit ("IMU") 158 or any one or more components thereof for inertial measurement selected from an accelerometer 160, a gyroscope 162, and a magnetometer 164 configured to provide positional-tracking data of the ultrasound probe 106 to the console 102 for stabilization of an image plane. The processor 116 is further configured to execute the logic 120 for processing the positional-tracking data for adjusting the distance of the activated ultrasonic transducers 149 from the target, the orientation of the activated ultrasonic transducers 149 to the target, or both the distance and the orientation of the activated ultrasonic transducers 149 with respect to the target to maintain the distance and the orientation of the activated ultrasonic transducers 149 with respect to the target when the ultrasound probe 106 is inadvertently moved with respect to the target.

It is appreciated that a medical device of a magnetizable material enables the medical device (e.g., the needle 112) to be magnetized by a magnetizer, if not already magnetized, and tracked by the ultrasound-imaging system 100 when the magnetized medical device is brought into proximity of the magnetic sensors 150 of the magnetic-sensor array 146 or inserted into the body of the patient *P* during an ultrasound-based medical procedure. Such magnetic-based tracking of the magnetized medical device assists the clinician in placing a distal tip thereof in a desired location, such as in a lumen of a blood vessel, by superimposing a simulated needle image representing the real-time distance and orientation of the needle 112 over an ultrasound image of the body of the patient *P* being accessed by the magnetized medical device. Such a medical device can be stainless steel such as SS 304 stainless steel; however, other suitable needle materials that are capable of being magnetized can be employed. So configured, the needle 112 or the like can produce a magnetic field or create a magnetic disturbance in a magnetic field detectable as magnetic signals by the magnetic-sensor array 146 of the ultrasound probe 106 so as to enable the distance and orientation of the magnetized medical device to be tracked by the ultrasound-imaging system 100 for dynamically adjusting the distance of the activated ultrasonic transducers 149, an orientation of the activated ultrasonic transducers 149, or both the distance and the orientation of the activated ultrasonic transducers 149 with respect to the magnetized medical device.

During operation of the ultrasound-imaging system 100, the probe head 114 of the ultrasound probe 106 is placed against skin of the patient *P.* An ultrasound beam 352 is produced so as to ultrasonically image a portion of a target area that may include a blood vessel beneath a surface of the skin of the patient P such as the blood vessel 351 of FIG. 3A. (*See* FIG. 3A.). The ultrasound beam defines an image plane 353 having a front side 353A consistent with a front side of the ultrasound probe 106 and an opposite facing back side 353B. The ultrasonic image of the blood vessel 351 can be depicted and stabilized on the display screen 104 of the ultrasound-imaging system 100 as shown in FIG. 3B.

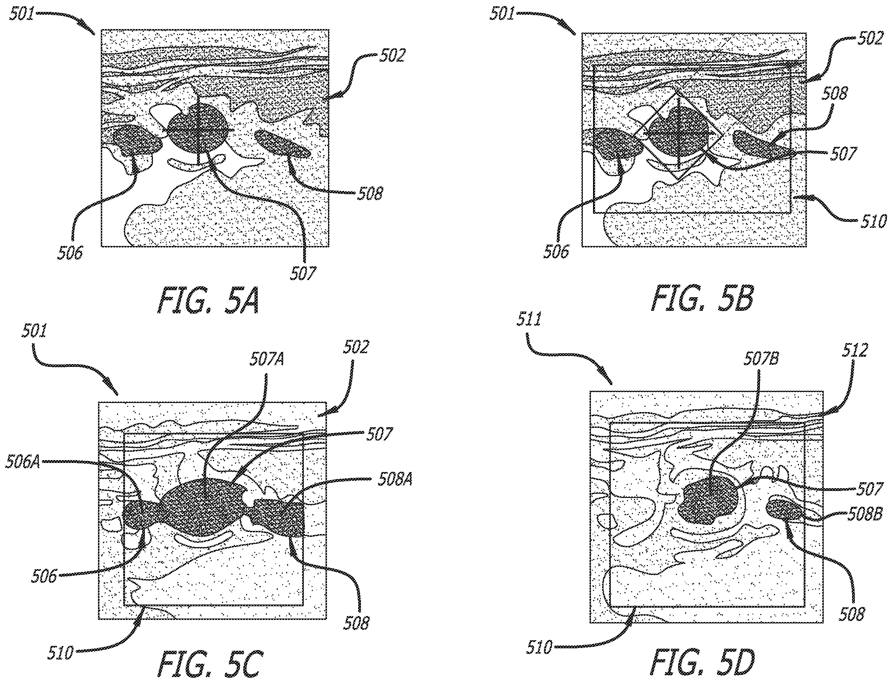

FIGS. 5A-5C illustrate an exemplary ultrasound image as may be obtained via the ultrasound probe 106 and as may be rendered the display 104. The ultrasound image 501 includes a predetermined target area 502 of the patient such as a subcutaneous portion of the patient's arm including blood vessels as further described below.

FIG. 5A illustrates the darker areas of the ultrasound image 501 having shapes that indicate specific anatomical structures of the subcutaneous tissue. Herein some darker areas are referred to as black holes, such as the black holes 506, 507, and 508. In some instances, the black holes may indicate blood vessels within the subcutaneous tissue or more specifically, the blood flow area of the blood vessels. In the illustrated embodiment, the logic 120 is configured to identify black holes within the ultrasound image that may be consistent with blood vessels and/or in some embodiments, nerves or nerve bundles. In the illustrated exemplary image 501, the logic 120 has identified the black holes 506, 507, and 508 as blood vessels and as such, the black holes 506, 507, and 508 may be referred to the blood vessels 506, 507, and 508 herein below.

In some embodiments, the logic 120 may identify one of the blood vessels 506, 507, and 508 as a target blood vessel in accordance with a predefined medical procedure. For example, the blood vessel 507 may be identified as a target blood vessel for receiving the needle 112 (see FIGS. 1, 3A). In some embodiments, identifying a black hole as a blood vessel may include comparing the ultrasound image with one or more corresponding ultrasound images stored in memory 118 (see FIG. 1) as further described below.

FIG. 5B further illustrates the ultrasound image 501. The ultrasound probe 106 is configured to detect motion of elements included in the ultrasound image 501 via doppler ultrasound. For example, the ultrasound probe 106 is configured to detect motion consistent with blood flow within the black holes (blood vessels) 506, 507, and 508. As such, the logic 120 may, based at least partially on doppler ultrasound, determine a blood flow within one or more of the black holes 506, 507, and 508 including a direction of the blood flow and, in some embodiments, a blood flow rate.

The logic 120 may further identify the black hole as a vein or alternatively as artery based on the determined direction of the blood flow within the black hole in relation to the image plane 353 of the ultrasound probe 106. For example, in further reference to FIG. 3A, the clinician in some instances may orient the ultrasound probe 106 consistent with a medical procedure, i.e., such that the front side of the ultrasound probe 106 and the corresponding front side 353A of the image plane 353 face upstream in relation to the venous flow. In such an instance, the logic 120 may identify a black hole having blood flow directed into the front side 353A of the image plane 353 as a vein. Conversely, in accordance with the same orientation of the ultrasound probe 106, the logic 120 may identify a black hole having blood flow directed out of the front side 353A of the image plane 353 as an artery.

In some embodiments, alternatively or in addition to direction of the blood flow, the logic 120 may identify a blood vessel as a vein or an artery based on a pulsatility of the blood flow. Typically, arterial blood flow is more pulsatile that venous blood flow. As such, the logic 120 may compare the pulsatility of the blood flow within a given blood vessel/black hole as measured via doppler ultrasound with a pulsatility limit stored in memory 118. In a first instance, where the measured pulsatility exceeds the pulsatility limit, the logic 120 may identify the blood vessel/black hole as an artery. Conversely, in a second instance, where the measured pulsatility is below the pulsatility limit, the logic 120 may identify the blood vessel/black hole as a vein.

In further embodiments, the logic 120 may identify a blood vessel as a vein or an artery based on the pulsatile motion of a blood vessel wall defining a cyclical change in black hole shape or size. In other words, pressure pules within a blood vessel due to pulsatile blood flow therethrough may cause the cross-sectional area (i.e., black hole area) of the blood vessel to expand and contract. As the arterial blood flow is more pulsatile that venous blood flow, the logic 120 may compare a magnitude of cyclical expansion and contraction of the blood vessel wall for a given blood vessel/black hole as measured via doppler ultrasound with an expansion and contraction magnitude limit stored in memory 118. In a first instance, where the measured magnitude of cyclical expansion and contraction exceeds the expansion and contraction magnitude limit, the logic 120 may identify the blood vessel/black hole as an artery. In a second instance, where the measured magnitude of cyclical expansion and contraction is below the expansion and contraction magnitude limit, the logic 120 may identify the blood vessel/black hole as a vein.

Generally, the ultrasound probe 106 may be configured to detect motion (e.g., blood flow) within a doppler ultrasound window 510. The doppler ultrasound window 510 may extend across all or a portion of the ultrasound image 501. In some embodiments, the logic 120 may automatically define the doppler ultrasound window 510 based on identified black holes within the ultrasound image 501. For example, the logic 120 may automatically define the doppler ultrasound window 510 to encompass one or more of the black holes 506, 507, and 508 within the ultrasound image 501.

In some embodiments, the logic 120 may automatically define the doppler ultrasound window 510 based partially on the comparison of the ultrasound image 501 with the corresponding ultrasound images stored in memory 118. For example, the corresponding ultrasound images may include a predefined doppler ultrasound window to be applied to the ultrasound image 501 and the logic 120 may define the doppler ultrasound window 510 according to the predefined doppler ultrasound window.

In some embodiments, the clinician may define the doppler ultrasound window 510 via input to the system 100 via an input device such as a computer mouse or other pointing device. In some embodiments, the input maybe facilitated via a GUI interface of the display 104. In other embodiments, the input may be facilitated via the control buttons 110 (see FIG. 1) on the ultrasound probe 106.

FIG. 5C further illustrates the ultrasound image 501. As stated above, the logic 120 may determine motion within the ultrasound window including blood flow with the black holes 506, 507, and 508. The doppler ultrasound process may operate in the background of the ultrasound imaging process. As shown in FIG. 5C, results of the doppler ultrasound process may include a direction of blood flow as illustrated by the direction indications 506A, 507A, and 508A which are shown in FIG. 5C for illustration purposes only, i.e., the direction indications 506A, 507A, and 508A may not be rendered on the display 104 along with the ultrasound image 501 because they obstruct the images of the black holes 506, 507, and 508. The direction indications 506A, 507A, and 508A may be based on the orientation of the image plane 353 (see FIG. 3A). In the illustrated embodiment, the direction indications 506A, 508A indicate a direction of the blood flow into the page (e.g., into the front side 353A of the image plane 353 of FIG. 3A). Conversely, the direction indication 507A indicates a direction of the blood flow out of the page (e.g., out of the front side 353A of the image plane 353 of FIG. 3A).

FIG. 5D further illustrates a second exemplary ultrasound image of a target area 512. In some instances, the target area 512 may be positioned adjacent the target area 502 of FIGS. 5A-5C, such as downstream of the target area 502, for example. In other instances, the target area 512 may be the same as the target area 502 with the ultrasound image 511 having been acquired at a different time than the ultrasound image 501, such as a subsequent time, for example. In the second exemplary image 511, the blood flow through the blood vessels is compromised (i.e., reduced). As shown, the size of the blood vessels 507 and 508 are reduced in relation to the areas of the blood vessels 507 and 508 of FIGS. 501A-501C. According to one example, the size of the black hole 506 of FIGS. 5A-5C is sufficiently reduced so that the logic 120 does not detect the black hole 506 in FIG. 5D. Similar to the FIG. 5C, direction indications 507B and 508B indicate the direction of the blood flow through blood vessels 507 and 508 consistent with the direction indications 507A and 508A of FIG. 5C.

In some embodiments, the logic 120 may compare the image 511 with corresponding images stored in memory 118 and as a result of the comparison determine the that the blood flows through the blood vessels 507 and 508 are reduced according to a size of the black holes. Alternatively, and/or in addition to the comparison of the image 511 with corresponding images stored in memory 118, the logic 120 may determine the reduction in blood flow via doppler ultrasound.

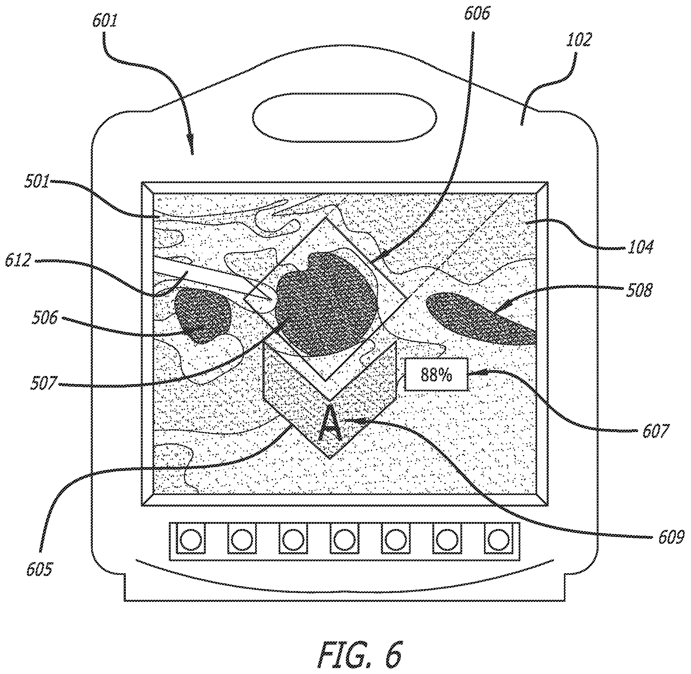

FIG. 6 illustrates an exemplary screenshot as may be rendered on the display 104. In the illustrated embodiment, the screenshot 601 includes the ultrasound image 501 and one or more notifications pertaining to the ultrasound image 501. The notifications may include one or more indicia relating to a target blood vessel, such as the blood vessel 507, for example. The notifications may include a condition of the blood flow within the blood vessel. The notifications may more specifically include a target indicium 606 indicating the identified black hole 507 as the target blood vessel pertaining to a defined medical procedure. The target indicium 606 is disposed outside the area of the black hole 507 so as to not obstruct the image of the black hole 507. In some embodiments, the target indicium 606 may include a border extending around the image of the black hole 507.

The notifications include a flow direction indicium 605 indicating the direction of the blood flow in relation to the black hole 507 as depicted in the ultrasound image 501. For example, in an instance where the direction of blood flow is out of the image plane (i.e., out of the screen of the display 104), the flow direction indicium 605 may include an arrow, pointer, or other shape pointing away from the black hole 507 indicating the direction of the blood flow out of the blood vessel 507 as shown in FIG. 6. Alternatively, in an instance where the direction of the blood flow is in the opposite direction, the flow direction indicium 605 may point toward the blood vessel 507. As may be appreciated by one of ordinary skill, the flow direction indicium 605 may include any shape, symbol, word, etc., suitable for indicating the direction of blood flow. In some embodiments, the target indicum 606 and the flow direction indicium 605 may be combined in a single indicum.

The notification may include a black hole identity indicator 609 for indicating the black hole as a vein or an artery. In some embodiments, the notifications may also include a confidence indicator 607 regarding the identification of the black hole 507 as an artery or alternatively as a vein. The confidence indicator 607 may be a number such as a percent probability or any other indicator suitable for communicating a confidence level of the identification.

In some embodiments, the screenshot 601 of the ultrasound image 501 may include an iconographic representation 612 of the needle 112 superimposed atop the ultrasound image 501 in accordance with some embodiments.

### Methods

Methods of the foregoing ultrasound-imaging systems include methods implemented in the ultrasound-imaging systems. For example, a method of the ultrasound-imaging system 100 includes a non-transitory CRM (e.g., EEPROM) having the logic 120 stored thereon that causes the ultrasound-imaging system 100 to perform a set of operations for ultrasound imaging when the logic 120 is executed by the processor 116 of the console 102. Such a method may, for description purposes, be referred to herein as activating operations, processing operations, and displaying operations. Any methods disclosed herein comprise one or more steps, actions or operations for performing the described method. The method includes operations (e.g., steps or actions) that may be interchanged with one another. In other words, unless a specific order of the operations is required for proper operation of the embodiment, the order and/or use of specific operations may be modified.

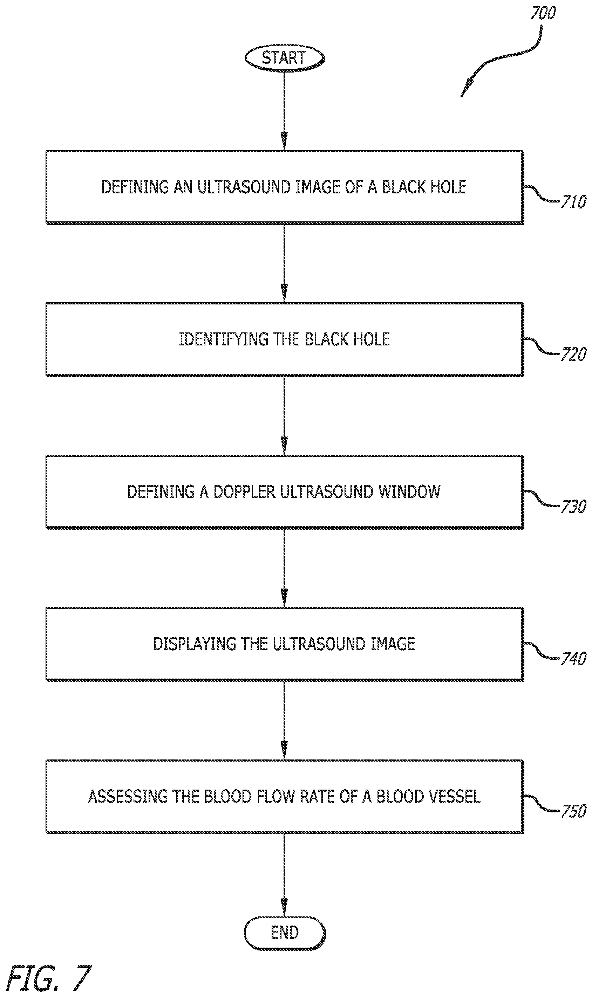

FIG. 7 is a flow chart illustrating an exemplary method of the system 100. The method 700 includes operations in accordance with a reference numbers 710-750. The method 700 generally includes defining an ultrasound image including a black hole (block 710).

Defining the ultrasound image may include activating include activating the ultrasonic transducers of the array of the ultrasonic transducers 148 of the ultrasound probe 106 communicatively coupled to the console 102. With the activating operation, the ultrasonic transducers 148 emit generated ultrasound signals into the patient *P,* receive reflected ultrasound signals from the patient *P,* and convert the reflected ultrasound signals into corresponding electrical signals for processing into ultrasound images. The activating operations can include activating an approximately linear subset of the ultrasonic transducers 148 of a 2-D array of the ultrasonic transducers 148. Alternatively, the activating operations can include activating a subset of the ultrasonic transducers 148 up to all the ultrasonic transducers 148 in the movable linear array of the ultrasonic transducers 148.

Defining the ultrasound image may further include analyzing ultrasound data to detect black holes within the ultrasound image. In doing so, the logic 120 may analyze ultrasound image data to search for and detect black holes within the predefined target area.

Upon detecting a black hole, the logic 120 may determine an identity of the black hole (block 720). The logic 120 may determine a blood flow condition within the black hole by obtaining doppler ultrasound data. The blood flow condition includes a presence or absence of blood flow. The processing operations includes determining the black hole to be blood vessel. The logic 120 detects, via doppler ultrasound, a blood flow within the black hole and thereby determine the black hole to be a blood vessel. The logic 120 may further determine a direction of the blood flow with respect to the image plane or more specifically with respect to an orientation of the image plane and thereby, determine the blood vessel to be vein or alternatively an artery. The logic 120 may also detect a non-flow condition of the black hole (i.e., absence of blood flow), and thereby, determine the black hole to be anatomical element other than a blood vessel such as a nerve or a bundle of nerves.

In some embodiments, the processing operations may further determine the blood vessel to be a vein or an artery based on a pulsatility of the blood flow. The logic 120 measure, via doppler ultrasound, a pulsatility of the blood flow within the black hole. As the arterial blood flow is generally more pulsatile than venous blood flow, the logic 120 may compare a measured pulsatility with a predefined pulsatility limit stored in memory. The logic 120 may thereby determine the blood vessel to be (1) an artery if the measured pulsatility exceeds the pulsatility limit or (2) a vein if the measured pulsatility is less than the pulsatility limit.

In some embodiments, the processing operations may further determine the blood vessel to be a vein or an artery based on a spatial positioning of the black hole within the ultrasound image. The logic 120 may compare the ultrasound image with one or more corresponding ultrasound images stored in memory. The logic 120 may more specifically compare the spatial positioning of the black hole within the ultrasound image with the spatial positioning of the corresponding black hole in the one or more corresponding ultrasound images, where the spatial positioning includes a subcutaneous depth of the black hole. As a result of the comparison, the logic 120 may identify the blood vessel as a vein or an artery.

In some embodiments, the processing operations may further include determining a confidence (e.g., a percent probability) for the determined identity of the black hole. The logic 120 may determine the confidence from the determined direction of blood flow, the measured pulsatility of the blood flow, the spatial positioning assessment of the black hole, or any combination thereof. The notification may include a number (e.g., a percent probability) or other confidence indication such as a low, medium, or high level indication of confidence.

The processing operations may further include establishing a doppler ultrasound window (block 730) within the ultrasound image, where the doppler ultrasound window defines a portion of the ultrasound window for assessing blood flow. In some embodiments, the logic 120 automatically may define the doppler ultrasound window to encompass one or more black holes detected within the ultrasound image. According to further embodiments, the logic 120 may receiving an input via an input device as may be entered by a clinician and the logic 120 may define the doppler ultrasound window based on the input. In other words, the clinician may select a portion of the ultrasound image as the doppler ultrasound window and the logic 120 may define the doppler ultrasound window in accordance with the selection.

The displaying operations further include rendering the ultrasound image on the display coupled with the console (block 740). The displaying operations may further include superimposing a visual notification atop the ultrasound image. The notification may indicate the direction of blood flow with respect to the image of the black hole. The logic 120 may cause the notification to be superimposed so as to not obstruct the black hole so that an image of a medical device may superimposed on the ultrasound image over the black hole. The logic 120 may specifically define a boundary or border surrounding the black hole in the ultrasound image. Having defined the boundary, the logic 120 may cause the notification to be superimposed the outside of the boundary. Having identified the black hole to be a vein or an artery, the logic 120 may include the identity of the black hole as a vein or an artery in the notification.

The processing operations may further include assessing the blood flow rate of the blood vessel (block 750). The logic 120 may determine a blood flow rate based on doppler ultrasound data. In some embodiments, the logic 120 may measure a velocity of blood flow across an area of the blood vessel and thereby determine a blood flow rate.

In some embodiments, the logic 120 may compare the measured blood flow rate with a range of blood flow rates for corresponding blood vessels stored in memory. As a result of the comparison, the logic 120 may determine that the blood flow within the blood vessel is compromised, e.g., low with respect to the range of blood flow rates. As a result of the comparison, the logic 120 may determine that the blood vessel is partially and/or totally occluded.

In some embodiments, the logic 120 may obtain a first blood flow rate measurement for the blood vessel and subsequently obtain a second blood flow rate measurement. The logic 120 may then comparing the second measurement with the first measurement and determine from the comparison that the blood flow rate is compromised when blood flow rate of the second measurement is less than the blood flow rate of the first measurement. In some instances, the second measurement may be obtained at a second location along the blood vessel different from a first location of the first measurement.

Other methods may include magnetic signal-related operations. The magnetic signal-related operations can include a converting operation. The converting operation includes converting magnetic signals from a magnetized medical device (e.g., the needle 112) with the magnetic-sensor array 146 of the ultrasound probe 106 into corresponding electrical signals. The processing operations further include processing the corresponding electrical signals of the magnetic signals with the processor 116 into distance and orientation information with respect to the predefined target area. The displaying operations further include displaying an iconographic representation of the medical device on the display screen 104. In some embodiments, the logic 120 may define the doppler ultrasound window based on the location of the iconographic representation of the medical device in relation to the ultrasound image.

Other methods may further include a number of optical signal-related operations in combination with further processing and displaying operations. The optical signal-related operations include emitting input optical signals, receiving reflected optical signals, and converting the reflected optical signals into corresponding electrical signals of the optical signals by the optical interrogator 154. The optical signal-related operations also include conveying the input optical signals from the optical interrogator 154 to the number of FBG sensors along the length of the optical-fiber stylet 156, as well as conveying the reflected optical signals from the number of FBG sensors back to the optical interrogator 154 with the optical-fiber stylet 156 disposed in a lumen of the medical device. The processing operation further include processing the corresponding electrical signals of the optical signals with the processor 116 into distance and orientation information with respect to the predefined target area. The displaying operations further include displaying an iconographic representation of a medical device on the display screen 104.

Other method operations can include a data-providing operation in combination with further processing operations. The data-providing operation includes providing positional-tracking data to the console 102 from the accelerometer 160, the gyroscope 162, the magnetometer 164, or a combination thereof of the ultrasound probe 106. The processing operations further include processing the positional-tracking data with the processor 116.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

## Claims

1. An ultrasound-imaging system (100), comprising:
an ultrasound probe (106) including an array of ultrasonic transducers (148), activated ultrasonic transducers (148) of the array of ultrasonic transducers (148) configured to emit generated ultrasound signals into a patient (P), receive reflected ultrasound signals from the patient (P), and convert the reflected ultrasound signals into corresponding electrical signals of the ultrasound signals for processing into ultrasound image data and doppler ultrasound data; and
a console (102) configured to communicate with the ultrasound probe (106), the console (102) including one or more processors (116) and a non-transitory computer-readable medium having stored thereon logic (120), when executed by the one or more processors (116), causes operations including:
detecting a black hole (506, 507, 508) within a predefined target area (502, 512) of the patient (P) based on ultrasound image data;
determining a blood flow condition within the black hole (506, 507, 508) based at least partially on doppler ultrasound data, wherein the blood flow condition includes a presence or absence of blood flow;
determining the black hole (506, 507, 508) to be a blood vessel when blood flow is detected within the black hole (506, 507, 508);
defining an ultrasound image of the predefined target area (502, 512) including an image (501, 511) of the black hole (506, 507, 508); and
superimposing a notification atop the ultrasound image (501, 511),
wherein the notification indicates the condition of blood flow within the black hole (506, 507, 508).

2. The ultrasound-imaging system (100) of claim 1, wherein:
the operations further include determining a direction of blood flow within the black hole (506, 507, 508) with respect to an image plane (353) of the ultrasound image (501, 511), and
the notification indicates the direction of blood flow with respect to the image of the black hole (506, 507, 508).

3. The ultrasound-imaging system (100) of either claim 1 or claim 2, wherein the notification does not obstruct the image of the black hole (506, 507, 508); and/or wherein the operations further include:
defining a boundary surrounding the image of the black hole (506, 507, 508); and
superimposing the notification outside of the boundary.

4. The ultrasound-imaging system (100) of any of the preceding claims, wherein the operations further include rendering the ultrasound image (501, 511) on a display (104) coupled with the console (102).

5. The ultrasound-imaging system (100) of any of the preceding claims, wherein the operations further include:
identifying the black hole (506, 507, 508) as a blood vessel;
identifying the blood vessel as a vein or alternatively as an artery, and
identifying the blood vessel as a vein or an artery based at least partially on the condition of the blood flow within the blood vessel;
optionally wherein the notification further indicates the identity of the blood vessel as a vein or as an artery.

6. The ultrasound-imaging system (100) of claim 5, wherein the operations further include identifying the blood vessel as a vein or alternatively as an artery based at least partially on the direction of the blood flow with respect to the image plane (353); and/or
wherein the operations further include:
measuring a pulsatility of the blood flow via doppler ultrasound; and
identifying the blood vessel as a vein or an artery based at least partially on the pulsatility measurement.

7. The ultrasound-imaging system (100) of any of claims 5-6, wherein the operations further include:
comparing the ultrasound image (501, 511) with one or more corresponding ultrasound images stored in memory (118), the comparison including a comparison of spatial positioning of the blood vessel within the ultrasound image (501, 511); and
identifying the blood vessel as a vein or as an artery at least partially as a result of the comparison.

8. The ultrasound-imaging system (100) of any of claims 5-7, wherein:
the operations further include determining a confidence for the identification of the blood vessel, and
the notification further indicates the confidence for the identification of the blood vessel.

9. The ultrasound-imaging system (100) of any of claims 5-8, wherein the operations further include:
measuring a blood flow rate within the blood vessel;
comparing the blood flow rate with a range of blood flow rates stored in memory (118); and
as a result of the comparison, determining that the blood flow rate within the blood vessel is compromised, optionally wherein the operations further include, as a result of the comparison, determining that the blood vessel is partially and/or totally occluded.

10. The ultrasound-imaging system (100) of any of claims 5-9, wherein the operations further include:
obtaining a first blood flow rate measurement for the blood vessel;
obtaining a second blood flow rate measurement for the blood vessel, the second measurement subsequent to the first measurement;
comparing the second measurement with the first measurement; and
as a result of the flow rate measurement comparison, determining that the blood flow rate within the blood vessel is compromised.

11. The ultrasound-imaging system (100) of any of the preceding claims, wherein the operations further include:
detecting a plurality of black holes (506, 507, 508) within the predefined target area (502, 512) of the patient (P) based on ultrasound image data;
determining a blood flow condition within each of the black holes (506, 507, 508) based at least partially on doppler ultrasound data; and
identifying each of the black holes (506, 507, 508) as a blood vessel or alternatively as one or more nerves,
wherein identifying a black hole (506, 507, 508) as one or more nerves includes determining a non-flow condition of blood within the respective black hole (506, 507, 508).

12. The ultrasound-imaging system (100) of any of the preceding claims, wherein the operations further include:
defining a doppler ultrasound window (510) extending at least partially across the ultrasound image (501, 511), the doppler ultrasound window (510) encompassing one or more black holes (506, 507, 508); and
determining the blood flow condition within each of the one or more black holes (506, 507, 508) encompassed by the doppler ultrasound window (510) based on doppler ultrasound data.

13. The ultrasound-imaging system (100) of claim 12, wherein defining the doppler ultrasound window (510) includes:
detecting the one or more black holes (506, 507, 508) based on the ultrasound image data; and
automatically defining the doppler ultrasound window (510) to encompass the one or more black holes (506, 507, 508); and/or
wherein defining the doppler ultrasound window (510) includes:
receiving an input via an input device of the system (100); and
defining the doppler ultrasound window (510) based on the input,
wherein the input includes a selected portion of the ultrasound image (501, 511).

14. The ultrasound-imaging system (100) of any of the preceding claims, the ultrasound probe (106) further comprising: an array of magnetic sensors (146) configured to convert magnetic signals from a magnetized medical device into corresponding electrical signals of the magnetic signals for processing by the processor (116) into distance and orientation information with respect to the predefined target area (502, 512) for rendering of an iconographic representation (612) of the medical device atop the ultrasound image (501, 511), optionally wherein defining the doppler ultrasound window (510) is based on a position of the iconographic representation (612) of the medical device atop the ultrasound image (501, 511).

15. A method of an ultrasound-imaging system (100) including a non-transitory computer-readable medium ("CRM") having executable logic (120) that cause the ultrasound-imaging system (100) to perform a set of operations for ultrasound imaging when the logic (120) is executed by a processor (116) of a console (102) of the ultrasound-imaging system (100), the method comprising:
activating ultrasonic transducers (148) of an array of ultrasonic transducers (148) of an ultrasound probe (106) communicatively coupled to the console (102), whereby the ultrasonic transducers (148) emit generated ultrasound signals into a patient (P), receive reflected ultrasound signals from the patient (P), and convert the reflected ultrasound signals into corresponding electrical signals of the ultrasound signals for processing into ultrasound images;
detecting a black hole (506, 507, 508) within a predefined target area (502, 512) of the patient (P) based on ultrasound image data;
determining a blood flow condition within the black hole (506, 507, 508) based at least partially on doppler ultrasound data, wherein the blood flow condition includes a presence or absence of blood flow;
determining the black hole (506, 507, 508) to be a blood vessel when blood flow is detected within the black hole (506, 507, 508);
defining an ultrasound image (501, 511) of the predefined target area (502, 512) including an image of the black hole (506, 507, 508); and
superimposing a notification atop the ultrasound image (501, 511),
wherein the notification indicates the condition of blood flow within the black hole (506, 507, 508).

## Patentansprüche

1. Ultraschallbildgebungssystem (100), umfassend:
eine Ultraschallsonde (106), einschließlich eines Arrays von Ultraschallwandlern (148), wobei die aktivierten Ultraschallwandler (148) des Arrays von Ultraschallwandlern (148) ausgebildet sind, um erzeugte Ultraschallsignale in einen Patienten (P) auszusenden, reflektierte Ultraschallsignale vom Patienten (P) zu empfangen und reflektierte Ultraschallsignale in entsprechende elektrische Signale der Ultraschallsignale zum Verarbeiten in Ultraschallbilddaten und Dopplerultraschalldaten umzuwandeln; und
eine Konsole (102), die dazu ausgebildet ist, mit der Ultraschallsonde (106) zu kommunizieren, wobei die Konsole (102) einen oder mehrere Prozessoren (116) und ein nicht transitorisches computerlesbares Medium umfasst, auf dem eine Logik (120) gespeichert ist, die, wenn sie von dem einen oder den mehreren Prozessoren (116) ausgeführt wird, Operationen bewirkt, einschließend:
Detektieren eines schwarzen Lochs (506, 507, 508) innerhalb eines vordefinierten Zielbereichs (502, 512) des Patienten (P), basierend auf Ultraschallbilddaten;
Bestimmen eines Blutflusszustands innerhalb des schwarzen Lochs (506, 507, 508), basierend zumindest teilweise auf Dopplerultraschalldaten, wobei der Blutflusszustand ein Vorhandensein oder Fehlen von Blutfluss einschließt;
Bestimmen, dass das schwarze Loch (506, 507, 508) ein Blutgefäß ist, wenn Blutfluss innerhalb des schwarzen Lochs (506, 507, 508) detektiert wird;
Definieren eines Ultraschallbilds des vordefinierten Zielbereichs (502, 512), einschließlich eines Bilds (501, 511) des schwarzen Lochs (506, 507, 508); und
Überlagern einer Benachrichtigung auf das Ultraschallbild (501, 511),
wobei die Benachrichtigung den Zustand des Blutflusses innerhalb des schwarzen Lochs (506, 507, 508) angibt.

2. Ultraschallbildgebungssystem (100) nach Anspruch 1, wobei:
die Operationen weiter das Bestimmen einer Richtung des Blutflusses innerhalb des schwarzen Lochs (506, 507, 508) in Bezug auf eine Bildebene (353) des Ultraschallbilds (501, 511) einschließen, und
die Benachrichtigung die Richtung des Blutflusses in Bezug auf das Bild des schwarzen Lochs (506, 507, 508) angibt.

3. Ultraschallbildgebungssystem (100) nach Anspruch 1 oder Anspruch 2, wobei die Benachrichtigung das Bild des schwarzen Lochs (506, 507, 508) nicht verdeckt; und/oder wobei die Operationen weiter Folgendes einschließen:
Definieren einer Begrenzung, die das Bild des schwarzen Lochs (506, 507, 508) umgibt; und
Überlagern der Benachrichtigung außerhalb der Begrenzung.

4. Ultraschallbildgebungssystem (100) nach einem der vorstehenden Ansprüche, wobei die Operationen weiter das Rendern des Ultraschallbilds (501, 511) auf einer Anzeige (104) einschließen, die mit der Konsole (102) gekoppelt ist.

5. Ultraschallbildgebungssystem (100) nach einem der vorstehenden Ansprüche, wobei die Operationen weiter Folgendes einschließen:
Identifizieren des schwarzen Lochs (506, 507, 508) als ein Blutgefäß;
Identifizieren des Blutgefäßes als eine Vene oder alternativ als eine Arterie, und
Identifizieren des Blutgefäßes als eine Vene oder eine Arterie, basierend zumindest teilweise auf dem Zustand des Blutflusses innerhalb des Blutgefäßes;
optional, wobei die Benachrichtigung weiter die Identität des Blutgefäßes als eine Vene oder als eine Arterie angibt.

6. Ultraschallbildgebungssystem (100) nach Anspruch 5, wobei die Operationen weiter das Identifizieren des Blutgefäßes als eine Vene oder alternativ als eine Arterie, basierend zumindest teilweise auf der Richtung des Blutflusses in Bezug auf die Bildebene (353), einschließen; und/oder
wobei die Operationen weiter Folgendes einschließen:
Messen einer Pulsatilität des Blutflusses mittels Dopplerultraschall; und
Identifizieren des Blutgefäßes als eine Vene oder eine Arterie, basierend zumindest teilweise auf der Pulsatilitätsmessung.

7. Ultraschallbildgebungssystem (100) nach einem der Ansprüche 5-6, wobei die Operationen weiter Folgendes einschließen:
Vergleichen des Ultraschallbilds (501, 511) mit einem oder mehreren entsprechenden Ultraschallbildern, die in einem Speicher (118) gespeichert sind, wobei der Vergleich einen Vergleich der räumlichen Positionierung des Blutgefäßes innerhalb des Ultraschallbilds (501, 511) einschließt; und
Identifizieren des Blutgefäßes als eine Vene oder als eine Arterie zumindest teilweise als Ergebnis des Vergleichs.

8. Ultraschallbildgebungssystem (100) nach einem der Ansprüche 5-7, wobei:
die Operationen weiter das Bestimmen einer Konfidenz für die Identifizierung des Blutgefäßes einschließen, und
die Benachrichtigung weiter die Konfidenz für die Identifizierung des Blutgefäßes angibt.

9. Ultraschallbildgebungssystem (100) nach einem der Ansprüche 5-8, wobei die Operationen weiter Folgendes einschließen:
Messen einer Blutflussrate innerhalb des Blutgefäßes;
Vergleichen der Blutflussrate mit einem Bereich von Blutflussraten, die in einem Speicher (118) gespeichert sind; und
als ein Ergebnis des Vergleichs, Bestimmen, dass die Blutflussrate innerhalb des Blutgefäßes beeinträchtigt ist, optional, wobei die Operationen weiter, als ein Ergebnis des Vergleichs, das Bestimmen einschließen, dass das Blutgefäß teilweise und/oder vollständig okkludiert ist.

10. Ultraschallbildgebungssystem (100) nach einem der Ansprüche 5-9, wobei die Operationen weiter Folgendes einschließen:
Erhalten einer ersten Blutflussratenmessung für das Blutgefäß;
Erhalten einer zweiten Blutflussratenmessung für das Blutgefäß, wobei die zweite Messung nach der ersten Messung erfolgt;
Vergleichen der zweiten Messung mit der ersten Messung; und
als ein Ergebnis des Vergleichs der Flussratenmessung, Bestimmen, dass die Blutflussrate innerhalb des Blutgefäßes beeinträchtigt ist.

11. Ultraschallbildgebungssystem (100) nach einem der vorstehenden Ansprüche, wobei die Operationen weiter Folgendes einschließen:
Detektieren einer Vielzahl von schwarzen Löchern (506, 507, 508) innerhalb des vordefinierten Zielbereichs (502, 512) des Patienten (P), basierend auf Ultraschallbilddaten;
Bestimmen eines Blutflusszustands innerhalb jedes der schwarzen Löcher (506, 507, 508), basierend zumindest teilweise auf Dopplerultraschalldaten; und
Identifizieren jedes der schwarzen Löcher (506, 507, 508) als ein Blutgefäß oder alternativ als einen oder mehrere Nerven,
wobei das Identifizieren eines schwarzen Lochs (506, 507, 508) als einen oder mehrere Nerven das Bestimmen eines Nichtflusszustands von Blut innerhalb des jeweiligen schwarzen Lochs (506, 507, 508) einschließt.

12. Ultraschallbildgebungssystem (100) nach einem der vorstehenden Ansprüche, wobei die Operationen weiter Folgendes einschließen:
Definieren eines Dopplerultraschallfensters (510), das sich zumindest teilweise über das Ultraschallbild (501, 511) erstreckt, wobei das Dopplerultraschallfenster (510) ein oder mehrere schwarze Löcher (506, 507, 508) umgibt; und
Bestimmen des Blutflusszustands innerhalb jedes der einen oder mehreren schwarzen Löcher (506, 507, 508), die von dem Dopplerultraschallfenster (510) umgeben sind, basierend auf Dopplerultraschalldaten.

13. Ultraschallbildgebungssystem (100) nach Anspruch 12, wobei das Definieren des Dopplerultraschallfensters (510) Folgendes einschließt:
Detektieren des einen oder der mehreren schwarzen Löcher (506, 507, 508), basierend auf den Ultraschallbilddaten; und
automatisches Definieren des Dopplerultraschallfensters (510), um das eine oder die mehreren schwarzen Löcher (506, 507, 508) zu umgeben; und/oder
wobei das Definieren des Dopplerultraschallfensters (510) Folgendes einschließt:
Empfangen einer Eingabe über eine Eingabevorrichtung des Systems (100); und
Definieren des Dopplerultraschallfensters (510), basierend auf der Eingabe,
wobei die Eingabe einen ausgewählten Abschnitt des Ultraschallbilds (501, 511) einschließt.

14. Ultraschallbildgebungssystem (100) nach einem der vorstehenden Ansprüche, wobei die Ultraschallsonde (106) weiter Folgendes umfasst: ein Array von Magnetsensoren (146), die dazu ausgebildet sind, magnetische Signale von einer magnetisierten medizinischen Vorrichtung in entsprechende elektrische Signale der magnetischen Signale zur Verarbeitung durch den Prozessor (116) in Distanz- und Orientierungsinformationen in Bezug auf den vordefinierten Zielbereich (502, 512) zum Rendern einer ikonografischen Darstellung (612) der medizinischen Vorrichtung auf dem Ultraschallbild (501, 511) umzuwandeln, optional, wobei das Definieren des Dopplerultraschallfensters (510), basierend auf einer Position der ikonografischen Darstellung (612) der medizinischen Vorrichtung auf dem Ultraschallbild (501, 511), erfolgt.

15. Verfahren eines Ultraschallbildgebungssystems (100), das ein nicht transitorisches computerlesbares Medium ("CRM") einschließt, das ausführbare Logik (120) aufweist, die bewirkt, dass das Ultraschallbildgebungssystem (100) einen Satz von Operationen für Ultraschallbildgebung ausführt, wenn die Logik (120) von einem Prozessor (116) einer Konsole (102) des Ultraschallbildgebungssystems (100) ausgeführt wird, wobei das Verfahren Folgendes umfasst:
Aktivieren von Ultraschallwandlern (148) eines Arrays von Ultraschallwandlern (148) einer Ultraschallsonde (106), die kommunikativ mit der Konsole (102) gekoppelt ist, wobei die Ultraschallwandler (148) erzeugte Ultraschallsignale in einen Patienten (P) emittieren, reflektierte Ultraschallsignale von dem Patienten (P) empfangen und die reflektierten Ultraschallsignale in entsprechende elektrische Signale der Ultraschallsignale zur Verarbeitung zu Ultraschallbildern umwandeln;
Detektieren eines schwarzen Lochs (506, 507, 508) innerhalb eines vordefinierten Zielbereichs (502, 512) des Patienten (P), basierend auf Ultraschallbilddaten;
Bestimmen eines Blutflusszustands innerhalb des schwarzen Lochs (506, 507, 508), basierend zumindest teilweise auf Dopplerultraschalldaten, wobei der Blutflusszustand ein Vorhandensein oder Fehlen von Blutfluss einschließt;
Bestimmen, dass das schwarze Loch (506, 507, 508) ein Blutgefäß ist, wenn Blutfluss innerhalb des schwarzen Lochs (506, 507, 508) detektiert wird;
Definieren eines Ultraschallbilds (501, 511) des vordefinierten Zielbereichs (502, 512), einschließlich eines Bilds des schwarzen Lochs (506, 507, 508); und
Überlagern einer Benachrichtigung auf das Ultraschallbild (501, 511),
wobei die Benachrichtigung den Zustand des Blutflusses innerhalb des schwarzen Lochs (506, 507, 508) angibt.

## Revendications

1. Système (100) d'imagerie par ultrasons, comprenant :
une sonde à ultrasons (106) incluant un réseau de transducteurs ultrasonores (148), les transducteurs ultrasonores activés (148) du réseau de transducteurs ultrasonores (148) étant configurés pour émettre des signaux à ultrasons générés chez un patient (P), recevoir des signaux à ultrasons réfléchis en provenance du patient (P) et convertir les signaux à ultrasons réfléchis en signaux électriques correspondants des signaux à ultrasons pour traitement en données d'image à ultrasons et données à ultrasons Doppler ; et
une console (102) configurée pour communiquer avec la sonde à ultrasons (106), la console (102) incluant un ou plusieurs processeurs (116) et un support non transitoire lisible par ordinateur sur lequel est stockée une logique (120), laquelle, lorsqu'elle est exécutée par les un ou plusieurs processeurs (116), provoque des opérations incluant :
la détection d'un trou noir (506, 507, 508) à l'intérieur d'une zone cible prédéfinie (502, 512) du patient (P) sur la base de données d'image à ultrasons ;
la détermination d'un état de flux sanguin à l'intérieur du trou noir (506, 507, 508) sur la base, au moins en partie, de données à ultrasons Doppler, dans lequel l'état de flux sanguin inclut une présence ou une absence de flux sanguin ;
la détermination que le trou noir (506, 507, 508) est un vaisseau sanguin lorsqu'un flux sanguin est détecté à l'intérieur du trou noir (506, 507, 508) ;
la définition d'une image à ultrasons de la zone cible prédéfinie (502, 512) incluant une image (501, 511) du trou noir (506, 507, 508) ; et
la superposition d'une notification sur l'image à ultrasons (501, 511),
dans lequel la notification indique l'état de flux sanguin à l'intérieur du trou noir (506, 507, 508).

2. Système (100) d'imagerie par ultrasons selon la revendication 1, dans lequel :
les opérations incluent en outre la détermination d'une direction de flux sanguin à l'intérieur du trou noir (506, 507, 508) par rapport à un plan d'image (353) de l'image à ultrasons (501, 511), et
la notification indique la direction de flux sanguin par rapport à l'image du trou noir (506, 507, 508).

3. Système (100) d'imagerie par ultrasons selon la revendication 1 ou la revendication 2, dans lequel la notification ne fait pas obstacle à l'image du trou noir (506, 507, 508) ; et/ou dans lequel les opérations incluent en outre :
la définition d'un contour entourant l'image du trou noir (506, 507, 508) ; et
la superposition de la notification à l'extérieur du contour.

4. Système (100) d'imagerie par ultrasons selon l'une quelconque des revendications précédentes, dans lequel les opérations incluent en outre le rendu de l'image à ultrasons (501, 511) sur un afficheur (104) couplé à la console (102).

5. Système (100) d'imagerie par ultrasons selon l'une quelconque des revendications précédentes, dans lequel les opérations incluent en outre :
l'identification du trou noir (506, 507, 508) comme étant un vaisseau sanguin ;
l'identification du vaisseau sanguin comme étant une veine ou, en variante, comme étant une artère, et
l'identification du vaisseau sanguin comme étant une veine ou une artère sur la base, au moins en partie, de l'état du flux sanguin à l'intérieur du vaisseau sanguin ;
facultativement, dans lequel la notification indique en outre l'identité du vaisseau sanguin en tant que veine ou en tant qu'artère.

6. Système (100) d'imagerie par ultrasons selon la revendication 5, dans lequel les opérations incluent en outre l'identification du vaisseau sanguin comme étant une veine ou, en variante, comme étant une artère sur la base, au moins en partie, de la direction du flux sanguin par rapport au plan d'image (353) ; et/ou
dans lequel les opérations incluent en outre :
la mesure d'une pulsatilité du flux sanguin par l'intermédiaire d'ultrasons Doppler ; et
l'identification du vaisseau sanguin comme étant une veine ou une artère sur la base, au moins en partie, de la mesure de la pulsatilité.

7. Système (100) d'imagerie par ultrasons selon l'une quelconque des revendications 5 et 6, dans lequel les opérations incluent en outre :
la comparaison de l'image à ultrasons (501, 511) avec une ou plusieurs images à ultrasons correspondantes stockées dans une mémoire (118), la comparaison incluant une comparaison du positionnement spatial du vaisseau sanguin à l'intérieur de l'image à ultrasons (501, 511) ; et
l'identification du vaisseau sanguin comme étant une veine ou comme étant une artère au moins en partie à la suite de la comparaison.

8. Système (100) d'imagerie par ultrasons selon l'une quelconque des revendications 5 à 7, dans lequel :
les opérations incluent en outre la détermination d'un niveau de confiance pour l'identification du vaisseau sanguin, et
la notification indique en outre le niveau de confiance pour l'identification du vaisseau sanguin.

9. Système (100) d'imagerie par ultrasons selon l'une quelconque des revendications 5 à 8, dans lequel les opérations incluent en outre :
la mesure d'un débit sanguin à l'intérieur du vaisseau sanguin ;
la comparaison du débit sanguin avec une plage de débits sanguins stockés dans la mémoire (118) ; et
à la suite de la comparaison, la détermination que le débit sanguin à l'intérieur du vaisseau sanguin est compromis, facultativement, dans lequel les opérations incluent en outre, à la suite de la comparaison, la détermination que le vaisseau sanguin est partiellement et/ou totalement obstrué.

10. Système (100) d'imagerie par ultrasons selon l'une quelconque des revendications 5 à 9, dans lequel les opérations incluent en outre :
l'obtention d'une première mesure de débit sanguin pour le vaisseau sanguin ;
l'obtention d'une deuxième mesure de débit sanguin pour le vaisseau sanguin, la deuxième mesure étant postérieure à la première mesure ;
la comparaison de la deuxième mesure avec la première mesure ; et
à la suite de la comparaison des mesures de débit, la détermination que le débit sanguin à l'intérieur du vaisseau sanguin est compromis.

11. Système (100) d'imagerie par ultrasons selon l'une quelconque des revendications précédentes, dans lequel les opérations incluent en outre :
la détection d'une pluralité de trous noirs (506, 507, 508) à l'intérieur de la zone cible prédéfinie (502, 512) du patient (P) sur la base de données d'image à ultrasons ;
la détermination d'un état de flux sanguin à l'intérieur de chacun des trous noirs (506, 507, 508) sur la base, au moins en partie, de données à ultrasons Doppler ; et
l'identification de chacun des trous noirs (506, 507, 508) comme étant un vaisseau sanguin ou, en variante, comme étant un ou plusieurs nerfs,
dans lequel l'identification d'un trou noir (506, 507, 508) comme étant un ou plusieurs nerfs inclut la détermination d'un état de non-écoulement de sang à l'intérieur du trou noir (506, 507, 508) respectif.

12. Système (100) d'imagerie par ultrasons selon l'une quelconque des revendications précédentes, dans lequel les opérations incluent en outre :
la définition d'une fenêtre d'ultrasons Doppler (510) s'étendant au moins partiellement à travers l'image à ultrasons (501, 511), la fenêtre d'ultrasons Doppler (510) englobant un ou plusieurs trous noirs (506, 507, 508) ; et
la détermination, sur la base de données à ultrasons Doppler, de l'état de flux sanguin dans chacun des un ou plusieurs trous noirs (506, 507, 508) englobés par la fenêtre d'ultrasons Doppler (510).

13. Système (100) d'imagerie par ultrasons selon la revendication 12, dans lequel la définition de la fenêtre d'ultrasons Doppler (510) inclut :
la détection des un ou plusieurs trous noirs (506, 507, 508) sur la base des données d'image à ultrasons ; et
la définition automatique de la fenêtre d'ultrasons Doppler (510) pour englober les un ou plusieurs trous noirs (506, 507, 508) ; et/ou
dans lequel la définition de la fenêtre d'ultrasons Doppler (510) inclut :
la réception d'une entrée via un dispositif d'entrée du système (100) ; et
la définition de la fenêtre d'ultrasons Doppler (510) sur la base de l'entrée,
dans lequel l'entrée inclut une partie sélectionnée de l'image à ultrasons (501, 511).

14. Système (100) d'imagerie par ultrasons selon l'une quelconque des revendications précédentes, la sonde à ultrasons (106) comprenant en outre : un réseau de capteurs magnétiques (146) configurés pour convertir des signaux magnétiques provenant d'un dispositif médical magnétisé en signaux électriques correspondants des signaux magnétiques pour traitement par le processeur (116) en informations de distance et d'orientation par rapport à la zone cible prédéfinie (502, 512) pour le rendu d'une représentation iconographique (612) du dispositif médical sur l'image à ultrasons (501, 511), facultativement dans lequel la définition de la fenêtre d'ultrasons Doppler (510) est basée sur une position de la représentation iconographique (612) du dispositif médical sur l'image à ultrasons (501, 511).

15. Procédé d'un système (100) d'imagerie par ultrasons incluant un support non transitoire lisible par ordinateur (« CRM ») présentant une logique exécutable (120) qui amène le système d'imagerie par ultrasons (100) à effectuer un ensemble d'opérations pour l'imagerie par ultrasons lorsque la logique (120) est exécutée par un processeur (116) d'une console (102) du système (100) d'imagerie par ultrasons, le procédé comprenant :
l'activation de transducteurs ultrasonores (148) d'un réseau de transducteurs ultrasonores (148) d'une sonde à ultrasons (106) couplée en communication à la console (102), de telle sorte que les transducteurs ultrasonores (148) émettent des signaux à ultrasons générés chez un patient (P), reçoivent des signaux à ultrasons réfléchis en provenance du patient (P) et convertissent les signaux à ultrasons réfléchis en signaux électriques correspondants des signaux à ultrasons pour traitement en images à ultrasons ;
la détection d'un trou noir (506, 507, 508) à l'intérieur d'une zone cible prédéfinie (502, 512) du patient (P) sur la base de données d'image à ultrasons ;
la détermination d'un état de flux sanguin à l'intérieur du trou noir (506, 507, 508) sur la base, au moins en partie, de données d'échographie Doppler, dans lequel l'état de flux sanguin inclut une présence ou une absence de flux sanguin ;
la détermination que le trou noir (506, 507, 508) est un vaisseau sanguin lorsqu'un flux sanguin est détecté à l'intérieur du trou noir (506, 507, 508) ;
la définition d'une image à ultrasons (501, 511) de la zone cible prédéfinie (502, 512) incluant une image du trou noir (506, 507, 508) ; et
la superposition d'une notification sur l'image à ultrasons (501, 511),
dans lequel la notification indique l'état de flux sanguin à l'intérieur du trou noir (506, 507, 508).
